# EUROPÄISCHE PATENTANMELDUNG

(11) **EP 2 314 608 A1**
(43) Veröffentlichungstag der Anmeldung: **27.04.2011**
(21) Anmeldenummer: 09174054.8
(22) Anmeldetag: 26.10.2009
(51) Int. Cl.: C07K 14/42, A61K 9/50

(54) **Stabilisierung von Biosensoren für in vivo Anwendungen**

(71) Anmelder: EyeSense AG, 4051 Basel (CH)
(72) Erfinder: Herbrechtsmeier, Peter, 61462 Königstein (DE); Müller, Achim, 63762 Grossostheim (DE); Knuth, Monika, 63741 Aschaffenburg (DE); Nikolaus, Katharina, 63739 Aschaffenburg (DE)
(74) Vertreter: Stößel, Matthias

(57) **Zusammenfassung**

Die vorliegende Erfindung betrifft Verfahren und Zubereitungen für die Stabilisierung isolierter Proteine. Insbesondere wird die Stabilisierung von Rezeptoren in biochemischen Sensoren offenbart.

## Beschreibung

Die vorliegende Erfindung betrifft Verfahren und Zubereitungen für die Stabilisierung isolierter Proteine. Insbesondere wird die Stabilisierung von Rezeptoren in biochemischen Sensoren offenbart.

Vielfach ist es für die technische Anwendung von Proteinen wünschenswert, diese von störenden Verunreinigungen zu befreien. Ein wichtiges Einsatzgebiet aufgereinigter Proteine sind biochemische Sensoren. Proteine, die als Rezeptoren biochemischer Sensoren verwendet werden, müssen eine hohe Affinität zum Analyten aufweisen. Um die Bindung des Analyten an den Rezeptor nachzuweisen wird der Rezeptor häufig modifiziert, um die Wechselwirkung von Analyt und Rezeptor messbar zu machen. Eine gezielte Modifizierung wird durch Verunreinigungen erschwert und vielfach unmöglich gemacht. Deswegen werden Proteine aus natürlichen Zellen und Geweben extrahiert und aufgereinigt.

Die Aufreinigung von Proteinen hat allerdings den Nachteil, dass die Stabilität des isolierten Proteins häufig geringer ist als seine Stabilität in der natürlichen Umgebung. Die Zugabe von Chaperonen, Aminosäuren, Proteinen (z.B. BSA), Polysacchariden oder Polysaccharidderivaten erhöht vielfach die Stabilität isolierter Proteine. Häufig werden nieder- oder hochmolekulare Zusätze verwendet, um das freie Volumen in der Lösung zu verringern ("molecular crowding"). Dabei können Proteine, Polysaccharide oder synthetische Polymere benutzt werden. Aus dem Stand der Technik sind insbesondere Polyethylenglycol, Ficoll, Dextran, Ribonuklease und bovines Serumalbumin bekannt. Der Effekt dieser Zugaben kann durch den Effekt des "ausgeschlossenen Volumens" erklärt werden (Chebotareva et al., 2004, Biochemical Effects of Molecular Crowding, Biochemistry (Moscow), 69: 1522-1536).

Aufgabe der Erfindung ist also die Bereitstellung von Verfahren und Zubereitungen, die eine verbesserte Stabilisierung isolierter Proteine ermöglichen. Die Aufgabe wird durch die Ausfiihrungsformen gelöst, die in den Patentansprüchen und nachfolgend beschrieben werden.

Die Erfindung betrifft ein Verfahren zur Bereitstellung einer ein isoliertes Protein stabilisierenden Zelllysatfraktion umfassend die Schritte
a) Gewinnung des Zelllysats aus den Zellen; und
b) Abtrennung des zu isolierenden Proteins aus dem Zelllysat.

Unter "Zellen" werden in dieser Anmeldung alle prokaryotischen oder eukaryotischen Zellen verstanden, die das zu isolierende Protein exprimieren. Dies schließt genetisch transformierte Zellen ein, in welchen das zu isolierende Protein rekombinant exprimiert wird. Die Zellen können als Einzelzellen einer prokaryotischen oder eukaryotischen Zellkultur vorliegen oder in Form einer Gewebeprobe eines tierischen oder pflanzlichen Organismus oder eines Pilzes.

Verfahren zur Gewinnung eines Zelllysats sind dem Fachmann gut bekannt. Die Zellen können im Rahmen des erfindungsgemäßen Verfahrens mit allen bekannten mechanischen oder nicht-mechanischen Aufschlussverfahren aufgeschlossen werden. Bevorzugte mechanische Aufschlussverfahren sind die Homogenisierung mit rotierenden Messern (bei tierischen Zellen), das Potter-Elvehjem-Verfahren, das Zermahlen der Zellen oder des Gewebes, das Zerreiben im Mörser mit Sand, Aluminiumoxid oder Glasperlen, der Zellaufschluss durch Kavitationskräfte bei Ultraschall und das Pressen einer Zellsuspension unter hohem Druck durch ein enges Ventil (z.B. in einer French Press). Der Fachmann weiß, dass bei mechanischen Aufschlussverfahren Wärme entstehen kann, so dass in vielen Fällen eine Temperaturregulierung notwendig ist, um eine Denaturierung der Proteine zu verhindern. Bevorzugte nicht-mechanische Aufschlussverfahren sind das wiederholte Einfrieren und Auftauen der Zellen, Behandlung der Zellen mit hypotonischen Löungen, die Behandlung mit Lysozym bei gram-positiven Bakterien, die Behandlung mit EDTA und anschließende Inkubation mit Lysozym bei gram-negativen Bakterien oder die Behandlung mit Toluen bei Hefen. Selbstverständlich können verschiedene der genannten Verfahren untereinander oder mit weiteren hier nicht genannten Aufschlussverfahren kombiniert werden.

Während des Aufschlusses müssen die Proteine vor schädlichen Einflüssen geschützt werden. Bevorzugt werden Kälte oder spezifische Inhibitoren verwendet um den Abbau der Proteine durch Proteasen zu verhindern. Zum Schutz von Thiolgruppen werden bevorzugt Reduktionsmittel, besonders bevorzugt Dithiothreitol oder Dithioerythol, eingesetzt. Zum Schutz vor Schwermetallionen ist Ethylendiamintetraessigsäure (EDTA) bevorzugt. Diese bindet auch zweiwertige Kationen, die Proteasen aktivieren können. Zur Verhinderung der Aggregation von Proteinen werden bevorzugt nicht-ionische Detergenzien verwendet.

Bevorzugt sind das Aufschlussverfahren und ggf. zu treffende Schutzmaßnahmen nicht nur darauf abgestimmt, dass das gewünschte Protein im Zelllysat in aktiver Form vorliegt. Auch die Bestandteile des Zelllysats, welche die Stabilisierung des Proteins vermitteln, dürfen durch das gewählte Aufschlussverfahren nicht in ihrer Funktion beeinträchtigt werden.

Vor ihrer praktischen Anwendung werden isolierte Proteine in vielen Fällen modifiziert. So werden Rezeptoren für den Einsatz in biochemischen Sensoren häufig markiert. Diese Markierungen erfolgen durch die kovalente Modifikation des Rezeptors beispielsweise mit Fluoreszenzfarbstoffen. Damit das modifizierte isolierte Protein, das stabilisiert werden soll, nicht durch das in der Zelllysatfraktion enthaltene nicht modifizierte Protein verdünnt wird, ist es bevorzugt, das zu isolierende Protein aus dem Zelllysat abzutrennen. Der Begriff "Abtrennung des zu isolierenden Proteins aus dem Zelllysat" bezeichnet die Aufreinigung des gewünschten Proteins. Als Ergebnis liegt wenigstens eine Zelllysatfraktion vor, die das Protein als isoliertes Protein enthält. Weiterhin liegt getrennt davon wenigstens eine Zelllysatfraktion vor, die das vorgenannte Protein nicht oder nur in geringem Anteil enthält. Das isolierte Protein ist von seinem natürlichen Kontext, d.h. von den Molekülen, mit denen es im Zelllysat vorlag, getrennt. Vorzugsweise liegt das isolierte Protein bezogen auf die Moleküle aus seiner natürlichen Umgebung zu wenigstens 50 %, wenigstens 75 %, wenigstens 80 %, wenigstens 90 %, wenigstens 95 % oder, besonders bevorzugt, zu wenigstens 99 % (Gewicht/Gewicht) rein vor. Die Zelllysatfraktion, die das isolierte Protein enthält, kann in beliebiger Menge Moleküle enthalten, die nicht der natürlichen Umgebung des besagten Proteins entstammen, sondern im Zuge der Aufreinigung oder nach der Aufreinigung zugefügt wurden. Ein Beispiel hierfür wären Bestandteile des verwendeten Puffers.

Verfahren zur Abtrennung eines zu isolierenden Proteins aus einem Zelllysat sind dem Fachmann bekannt. Bevorzugte Verfahren sind Fällung und differentielle Solubilisierung, Ultrazentrifugation, chromatographische Verfahren und Elektrophorese.

Die Fällung von Proteinen wird bevorzugt durch die Zugabe von Ammoniumsulfat in steigenden Konzentrationen erreicht. Proteinfraktionen verschiedener Löslichkeit fallen bei diesem Verfahren in Abhängigkeit von der erreichten Ammoniumsulfatkonzentration nacheinander aus und können abgetrennt werden. Gleichfalls bevorzugt ist die Ausfällung des Proteins durch Ansäuerung des Medium mit einem geeigneten Puffer.

Ultrazentrifugation beruht auf dem Prinzip, dass im durch die Zentrifugation erzeugten Schwerefeld Partikel umso schneller sedimentieren, je dichter und kompakter sie sind. Als Folge des Gleichgewichts zwischen Zentrifugalkraft und Auftrieb, das sich während einer ausreichend langen Zentrifugation einstellt, reichern sich die einzelnen Proteine an den Stellen im Gefäß an, an denen sich Auftrieb und Zentrifugalkraft gegenseitig ausgleichen. Die Verwendung von Sucrose-Gradienten kann diesen Prozess erleichtern.

Die chromatografische Isolierung von Proteinen beruht auf dem Prinzip, dass die in einer mobilen Phase, bevorzugt einem Puffer, gelösten Proteine über eine stationäre Phase wandern. Die Phasen sind so gewählt, dass die unterschiedlich starke Interaktion einzelner Proteine aus dem Proteingemisch mit der festen Phase zu unterschiedlich langen Laufzeiten der einzelnen Proteinfraktionen über die stationäre Phase führt. Auf welche Art Proteine und stationäre Phase interagieren, hängt von der Art der Chromatographie ab. Bevorzugte chromatographische Verfahren zur Isolierung von Proteinen sind Größenausschlusschromatographie (basierend auf der unterschiedlichen Größe der aufzutrennenden Proteine), Affinitätschromatographie (basierend auf der Fähigkeit einzelner Proteine, spezifisch an das Säulenmaterial zu binden), Ionenaustauschchromatographie (basierend auf den unterschiedlichen Isoelektrischen Punkten verschiedener Proteine) und Umkehrphasenchromatographie (basierend auf der unterschiedlichen Hydrophobizität verschiedener Proteine).

Der Begriff "isoliertes Protein" bezeichnet im Rahmen dieser Patentanmeldung beliebige Proteine, an deren Isolierung in funktionalem Zustand Interesse besteht. Im Folgenden wird der Begriff "gewünschtes Protein" synonym verwendet. Isolierte Proteine im Sinne der vorliegenden Anmeldung umfassen bevorzugt Enzyme, Rezeptoren, modulatorische Proteine, Transkriptionsfaktoren, Zytoskelettproteine, Bindeproteine und Membrantransporter, besonders bevorzugt Rezeptoren. Ganz besonders bevorzugt ist das isolierte Protein Concanavalin A. Die isolierten Proteine können im Rahmen des Verfahrens als Reinstoffe oder partiell isoliert vorliegen. Bevorzugt umfassen isolierte Proteine oder Präparationen von isolierten Proteinen weniger als 50 %, weniger als 25 %, weniger als 10 %, weniger als 5 %, weniger als 1 % oder weniger als 0,5 % Protein oder Protein und zelluläre Bestandteile als Verunreinigung.

Unter "Enzym" wird jedes Protein verstanden, das in der Lage ist, eine chemische Reaktion zu katalysieren. Dabei spielt es keine Rolle, ob im Rahmen der chemischen Reaktion andere Moleküle umgewandelt werden oder ob das Enzym autokatalytisch wirkt.

Ein "Rezeptor" ist jedes Protein, das in der Lage ist, ein Molekül, den Liganden, spezifisch zu binden und auf diese Bindung mit einer Konformationsveränderung oder einer Aktivitätsänderung zu reagieren. Auch Proteine, die in ihrem natürlichen Zustand keine Reaktion auf die Bindung eines Liganden zeigen, können chemisch modifiziert werden, so dass die Bindung eines Liganden zu messbaren Veränderungen des Proteins führt. Weiterhin sind durch die Kombination eines fluoreszenzmarkierten Rezeptors mit einem ebenfalls fluoreszenmarkierten Liganden kompetetive Assays möglich. In der Abwesenheit von unmarkiertem Liganden in der Probe bindet nur der fluoreszenzmarkierte Ligand an den Rezeptor. Die räumliche Nähe der beiden Fluoreszenzfarbstoffe beeinflusst ihre Lichtemission. Ist nun in einer Probe unmarkierter Ligand vorhanden, so verdrängt der unmarkierte Ligand den fluoreszenzmarkierten Liganden vom Rezeptor und verändert so die Lichtemission des Systems. Die Veränderung der Lichtemission des Systems hängt somit von der Menge an nicht markiertem Liganden ab, der mit der Probe in das System eingebracht wird und markierte Liganden von den Rezeptoren verdrängt. Spezifische Bindung bedeutet, dass der Ligand mit signifikant höherer Affinität gebunden wird als andere Substanzen. Vorzugsweise wird der Ligand mit wenigstens 10-fach, 100-fach, 1000-fach, 10.000-fach oder 100.000-fach höherer Affinität gebunden als andere Liganden. Liganden sind bevorzugt Ionen, kleine Moleküle, Nukleinsäuren (DNA oder RNA in einzel- oder doppelsträngiger Form) oder andere Proteine beliebiger Länge. Kleine Moleküle können zu jeder bekannten Klasse von Molekülen gehören. Sie sind bevorzugt Lipide, Fettsäuren, Purine, Pyrimidine, Zucker, Alkaloide, Aminosäuren, biogene Amine, Isoprenoide oder Steroide. Besonders bevorzugt ist das kleine Molekül ein Zucker, ganz besonders bevorzugt Glucose. Besonders bevorzugt ist der Rezeptor ein Lektin, ganz besonders bevorzugt Concanavalin A.

Lektine sind Proteine, die in der Lage sind, Kohlehydratstrukturen spezifisch zu binden. Sie sind an vielfältigen molekularen und zellulären Erkennungsprozessen in Tieren, Pflanzen und Bakterien beteiligt und haben keine enzymatische Funktion. Viele Lektine sind postranslational durch Glykosylreste modifiziert.

Concanavalin A ist ein Lektin, das α-D-Glucose und ähnliche Zucker binden kann, ohne diese enzymatisch umzusetzen. Das Monomer besteht aus 237 Aminosäuren und enthält Mangan und Calcium. Bei neutralem pH bildet es ein Tetramer, das im sauren Bereich in zwei Dimere zerfällt. Es kommt in besonders hoher Konzentration in der Schwertbohne *Canavalia ensiformis* vor. Concanavalin A hat bevorzugt die durch SEQ ID NO: 3 definierte Aminosäuresequenz.

Als "modulatorische Proteine" werden in dieser Anmeldung solche Proteine bezeichnet, die mit anderen Proteinen interagieren und dadurch die Aktivität dieser Proteine verändern. Die Folge kann eine reduzierte Aktivität des Interaktionspartners sein (Inhibition) oder eine gesteigerte Aktivität (Aktivierung). Der Bindungspartner des Modulators ist bevorzugt ein Enzym. In diesem Fall beeinflusst die Bindung des Modulators die Affinität für das Substrat oder die Geschwindigkeit der enzymatischen Umsetzung des Substrats. Ebenfalls bevorzugt ist der Bindungspartner ein Rezeptor. In diesem Fall kann der Modulator agonistisch wirken, d.h. die durch Bindung an den Rezeptor diesen aktivieren oder der Modulator kann als Antagonist an den Rezeptor binden, ohne diesen dabei zu aktivieren. Weiterhin bevorzugt ist der Bindungspartner ein Transkriptionsfaktor. In diesem Fall beeinflusst die Bindung des Modulators die Fähigkeit des Transkriptionsfaktors, seinerseits die Genexpression zu verändern.

Ein "Transkriptionsfaktor" im Sinne dieser Anmeldung ist ein Protein, dessen Aktivität darin besteht, die Expression eines oder mehrerer Gene in der Zelle zu fördern oder zu inhibieren.

"Bindeproteine" sind Proteine, die in der Lage sind, andere Moleküle (Liganden) spezifisch zu binden. Spezifische Bindung bedeutet, dass der Ligand mit signifikant höherer Affinität gebunden wird als andere Substanzen. Vorzugsweise wird der Ligand mit wenigstens 10-fach, 100-fach, 1000-fach, 10.000-fach oder 100.000-fach höherer Affinität gebunden als andere Liganden.

Unter dem Begriff "Membrantransporter" werden solche Proteine verstanden, die die Passage anderer Moleküle durch die Zellmembran ermöglichen oder erleichtern. Diese Proteine sind in der Zellmembran lokalisiert.

Zytoskelettproteine sind Proteine, die die räumliche Struktur einer Zelle stabilisieren und in Kombination mit Motorproteinen zelluläre Bewegungsvorgänge vermitteln. Zytoskelettproteine sind bevorzugt Actin, Intermediärfilamente und Mikrotubuli. Mit Actinfilamenten und Mikrotubuli assoziierte Motorproteine sind bevorzugt Kinesin, Dynein und Myosin.

Der Begriff "Stabilisierung" bezeichnet die Aufrechterhaltung der Struktur und/oder der Funktion eines isolierten Proteins. Da die Struktur eines Proteins wesentliche Voraussetzung für dessen Funktion ist, führt eine Stabilisierung der Proteinstruktur vorzugsweise zu einer Aufrechterhaltung der Funktion des Proteins. Die zu stabilisierende Struktur des Proteins ist vorzugsweise jene Struktur, die es in seiner natürlichen Umgebung, d.h. im Gewebe oder in der Zelle hat. Die erfindungsgemäße Zubereitung stabilisiert vorzugsweise die Sekundär-, Tertiär- oder Quartärstruktur des isolierten Proteins. Unter Stabilisierung wird im Rahmen dieser Erfindung die Stabilisierung eines statistisch signifikanten Anteils der Moleküle des isolierten Proteins verstanden. So soll die Wiederfindungsrate an funktionalem isoliertem Protein nach Verabreichung der erfindungsgemäßen Zubereitung vorzugsweise zumindest 50 %, zumindest 75 %, zumindest 80 % zumindest 90 %, zumindest 95 % oder, besonders bevorzugt, zumindest 99 % betragen. Die Wiederfindungsrate kann daher bestimmt werden als Anteil funktionaler Moleküle des isolierten Proteins an der Gesamtmenge der Moleküle des isolierten Proteins nach Zugabe der erfindungsgemäßen Zubereitung und Lagerung. Bevorzugt werden zur Messung der Wiederfindungsrate die Aktivitäten der das isolierte Protein enthaltenden Zubereitung unmittelbar vor oder nach Zugabe der erfindungsgemäßen Zubereitung und zu einem weiteren Zeitpunkt verglichen. Außerdem erfolgen Vergleiche mit Zubereitungen des isolierten Proteins ohne Zugabe der erfindungsgemäßen Zubereitung zu gleichen Zeitpunkten.

Die vorliegende Erfindung ermöglicht vorteilhaft die verbesserte Stabilisierung isolierter Proteine, insbesondere Rezeptoren. Wie Beispiel 5 zeigt, wird Concanavalin A durch die nichtbindende Zelllysatfraktion aus den Samen von *Canavalia ensiformis* besser stabilisiert als durch bovines Serumalbumin, das im Stand der Technik als Stabilisator für Proteine bekannt ist. Ein weiterer überraschender Vorteil der erfindungsgemäßen, ein isoliertes Protein stabilisierenden Zelllysatfraktion ist die Möglichkeit, diese zu autoklavieren, ohne dass ihre Aktivität beeinträchtigt wird. Für Anwendungen der erfindungsgemäßen, ein isoliertes Protein stabilisierenden Zelllysatfraktion im medizinischen Bereich ist dieses ein erheblicher Vorteil, da so die Keimfreiheit der Zelllysatfraktion sichergestellt werden kann.

In einer bevorzugten Ausfiihrungsform der vorliegenden Erfindung werden das isolierte Protein und die das isolierte Protein stabilisierende Zelllysatfraktion aus dem Samen der Schwertbohne (*Canavalis ensiformis*) gewonnen.

In einer besonders bevorzugten Ausführungsform der vorliegenden Erfindung enthält die das isolierte Protein stabilisierende Zelllysatfraktion Präcanavalin. Wenn die das zu isolierende Protein produzierende Zelle in ihrer natürlichen Form kein oder nicht ausreichend Präcanavalin enthält, ist es bevorzugt, in dieser Zelle Präcanavalin rekombinant zu exprimieren. Die Zugabe von Präcanavalin zu einem Zelllysat ist ebenfalls bevorzugt.

Der Begriff "Präcanavalin" bezeichnet ein im Samen der Schwertbohne (*Canavalia ensiformis*) enthaltenes Protein. Präcanavalin-Monomere zeigen in der Gelelektrophorese unter denaturierenden Bedingungen ein Molekulargewicht von 49.000 Dalton. Durch Trypsin wird Präcanavalin in zwei Peptide von 24.000 bzw 25.000 Dalton Molekulargewicht gespalten. Unter Bedingungen, die eine Kristallisation dieser Spaltprodukte erlauben, wird das größere der beiden Peptide ein weiteres Mal durch Trypsin gespalten (Campbell Smith et 1., 1982, Biochemical Characterization of Canavalin, the Major Storage Protein of Jack Bean, Plant Physiology, 70: 1199-1209). Vorzugsweise hat Canavalin eine Aminosäuresequenz wie in SEQ ID NO: 2 definiert. In einer bevorzugten Ausführungsform der vorliegenden Erfindung weist das Präcanavalin eine Sequenzidentität von wenigstens 60 %, 70 %, 80 %, 90 %, 95 %, 97 %, 98 % oder 99 % zu der durch SEQ ID NO: 2 definierten Aminosäuresequenz auf. Vorzugsweise wird Präcanavalin durch ein Polynukleotid mit der durch SEQ ID NO: 1 definierten Sequenz kodiert. Ein Verfahren zur Bereitstellung von aufgereinigtem Präcanavalin aus der Schwertbohne ist in Beispiel 1 beschrieben.

In einer weiteren bevorzugten Ausfiihrungsform der vorliegenden Erfindung wird die ein isoliertes Protein stabilisierende Zelllysatfraktion weiter aufgearbeitet.

Diese Aufarbeitung besteht bevorzugt in der Zugabe von Protease-Inhibitoren und/oder der Bindung freier Metallionen durch Chelatoren. Weiterhin ist die Sterilisierung der das Protein stabilisierenden Zelllysatfraktion bevorzugt. Ganz besonders bevorzugt besteht die Aufarbeitung in der Anreicherung einer Subpopulation von das isolierte Protein besonders wirksam stabilisierenden Bestandteilen in der Zelllysatfraktion. Ebenfalls ganz besonders bevorzugt ist die Abtrennung von störenden Bestandteilen aus der Zelllysatfraktion. Eine solche Auftrennung der das Protein stabilisierenden Zelllysatfraktion wird eingesetzt, um aus besagter Zelllysatfraktion solche Moleküle zu entfernen, die die technische Anwendung des Proteins behindern. Zur Isolierung einer Subpopulation von Molekülen aus der das Protein stabilisierenden Zelllysatfraktion können alle dem Fachmann bekannten Verfahren zur Auftrennung von Molekülgemischen verwendet werden. Bevorzugt werden die Trennungsverfahren eingesetzt, die oben für die Aufreinigung von Proteinen beschrieben wurden.

Im Rahmen der der vorliegenden Erfindung zugrunde liegenden Studie hat sich herausgestellt, dass Präcanavalin besonders gut dazu geeignet ist, isolierte Proteine zu stabilisieren. Deswegen besteht die weitere Aufarbeitung der ein isoliertes Protein stabilisierenden Zelllysatfraktion vorzugsweise in der Anreicherung von Präcanavalin.

Präcanavalin wird bevorzugt durch ein Verfahren angereichert, welches die folgenden Schritte umfasst:
1. Suspension von Schwertbohnenmehl in geeignetem Puffer;
2. Chromatografische Abtrennung des Concanavalin A mit einer Superdex 200- Säule. Das Präcanavalin befindet sich in der nichtbindenden Fraktion;
3. Dialyse der nichtbindenden Fraktion gegen Wasser;
4. Lyophilisierung der nichtbindenden Fraktion;
5. Versetzen der nichtbindenden Fraktion mit saurem Fällungspuffer, bevorzugt 50 mM Na-Acetat, pH 4,4 und anschließende Zentrifugation;
6. Waschen des Sediments mit Aqua dest. und anschließende Zentrifugation;
7. Das verbleibende Pellet wird in geeignetem Puffer, bevorzugt 1 % (Gewicht/Gewicht) NaCl und 0,1 % (Gewicht/Gewicht) K₂HPO₄, pH 7,0, aufgenommen und zentrifugiert;
8. Nach der Zentrifugation wird der Überstand gesammelt;
9. Das nach der Zentrifugation verbleibende Sediment wird in Kochsalzlösung, vorzugsweise 5 % (Gewicht/Gewicht) aufgenommen und erneut zentrifugiert;
10. Der in Schritt 9 erhaltene Überstand wird mit dem Überstand aus Schritt 8 vereinigt;
11. Dialyse der vereinigten Überstände gegen Aqua dest;
12. Aufkonzentration des Präcanavalins durch Säure-Base-Extraktion, bevorzugt durch Fällung mit 1 N Essigsäure, pH 5.1 und Aufnahme des Sediments in 0,01 N NaOH, wobei der pH nach der Aufnahme in der Base nicht mehr als 8,0 beträgt;
13. Dialyse gegen den für die anschließende Ionenaustauschchromatographie vorgesehenen Auftragspuffer, bevorzugt 20 mM Tris, 100 mM NaCL, pH 7,2;
14. Ionenaustauschchromatographie, bevorzugt mit einer Säule aus DEAE Sepharose FF und dem in Schritt 13 genannten Auftragspuffer, Elution bevorzugt mit steigendem Salzgradienten;
15. Identifikation der reinsten Fraktionen, bevorzugt mit SDS-PAGE, Vereinigung dieser Fraktionen;
16. Dialyse gegen Aqua dest.; und
17. Gefriertrocknung des Präcanavalins

Besonders bevorzugt wird Präcanavalin durch ein vereinfachtes Verfahren angereichert, welches die Schritte umfasst:
1. Suspension von Schwertbohnenmehl in geeignetem Puffer;
2. Ionenaustauschchromatographie, bevorzugt mit einer Säule aus DEAE-Sepharose FF, Elution bevorzugt mit steigendem Salzgradienten;
3. Identifikation der reinsten Fraktionen, bevorzugt mit SDS-PAGE, Vereinigung dieser Fraktionen;
4. Dialyse gegen Aqua dest.; und
5. Gefriertrocknung des Präcanavalins.

Eine weiter bevorzugte Ausführungsform der vorliegenden Erfindung betrifft die Verwendung einer Zubereitung enthaltend Präcanavalin zur Stabilisierung eines isolierten Proteins. Besonders bevorzugt ist die Verwendung der vorgenannten Zubereitung für die Stabilisierung von Concanavalin A.

Eine "Zubereitung enthaltend Präcanavalin" liegt vorzugsweise in flüssiger oder fester Form vor. Vorzugsweise enthält die Zubereitung außer Präcanavalin noch weitere Bestandteile aus der zellulären Umgebung des isolierten Proteins. Gleichfalls bevorzugt enthält die Zubereitung Chaperone, bovines Serumalbumin, und/oder kompatible Solute. Weiterhin bevorzugt enthält die Zubereitung organische oder anorganische Puffersubstanzen. Auch Konservierungsmittel, welche das Wachstum von Bakterien und Pilzen unterdrücken, und Benetzungsmittel sind als Bestandteile der erfindungsgemäßen Zubereitung bevorzugt. Der Gewichtsanteil von Präcanavalin an der Trockenmasse der erfindungsgemäßen Zubereitung beträgt vorzugsweise wenigstens 30 %, 40 %, 50 %, 60 %, 70 %, 80 %, 90 %, 95 %, 97 %, 98 %, 99 % oder 99,5 %.

In einer bevorzugten Ausführungsform wird das in der erfindungsgemäßen Zubereitung enthaltene Präcanavalin rekombinant hergestellt.

Verfahren zur rekombinanten Herstellung von Proteinen sind dem Fachmann gut bekannt. Eine Nukleinsäuresequenz, die für Präcanavalin kodiert wird in einen Expressionsvektor eingebracht, der die Expression des Präcanavalins in dem gewählten Wirtsorganismus ermöglicht. Hierfür ist es bevorzugt, dass die Nukleinsäuresequenz, die für Präcanavalin kodiert unter Kontrolle einer geeigneten regulatorischen Sequenz steht. Geeignete regulatorische Sequenzen sind beispielsweise der lac-, trp- oder tac-Promotor für *E. coli,* der AOX1- oder GAL-1 Promotor für Hefen oder der CaMV-Promotor für Pflanzen. Für den Einsatz in tierischen Zellen sind der CMV-, SV40-, oder der RSV-promoter (Rous sarcoma virus) sowie der CMV-enhancer, der SV40-enhancer oder ein Globin-Intron bevorzugt. Bevorzugte Expressionsvektoren sind Plasmide, Phagen, retrovirale Vektoren und künstliche Chromosomen. Bevorzugte Wirtsorganismen für die rekombinante Herstellung von Präcanavalin sind Pflanzen, Prokaryoten und Pilze oder auch Säugetiere. Plasmide werden bevorzugt durch Elektroporation, Fällung mit Calciumphosphat oder Rubidiumchlorid oder durch Hitzeschock in die Wirtszelle eingebracht. Bevorzugte Verfahren für die Transformation von Pflanzenzellen sind die ballistische Inokulation und die durch *Agrobacterium tumefaciens* vermittelte Transformation.

Die vorliegende Erfindung betrifft weiterhin ein Verfahren zur Stabilisierung eines isolierten Proteins umfassend die Zugabe einer erfindungsgemäßen Präcanavalin enthaltenden Zubereitung zu dem isolierten Protein.

Die Zugabe der erfindungsgemäßen Präcanavalin enthaltenden Zubereitung zu dem gewünschten Protein ermöglicht die exakte Dosierung der erfindungsgemäßen Zubereitung. So kann eine definierte und reproduzierbare Stabilisierung des gewünschten Proteins erreicht werden. Bevorzugt wird die getrocknete Zubereitung als Feststoff in die das gewünschte Protein enthaltende Lösung gegeben und darin gelöst. Gleichfalls bevorzugt wird eine Stammlösung der erfindungsgemäßen Zubereitung der das gewünschte Protein enthaltende Lösung zugefügt. Besonders bevorzugt ist der Einbau der erfindungsgemäßen, Prävanavalin enthaltenden Zubereitung in Hydrogelpartikel, ganz besonders bevorzugt Alginatkugeln, wobei die Hydrogelparttikel auch das isolierte Protein enthalten.

In einer bevorzugten Ausfiihrungsform des erfindungsgemäßen Verfahrens ist das isolierte Protein ein Rezeptor, stärker bevorzugt ein Lektin und noch stärker bevorzugt Concanavalin A.

Weiterhin betrifft die vorliegende Erfindung die Verwendung der erfindungsgemäßen ein isoliertes Protein stabilisierenden Zelllysatfraktion zur Stabilisierung eines isolierten Proteins, wobei besagtes isoliertes Protein Bestandteil eines biochemischen Sensors ist.

Ein "biochemischer Sensor" enthält ein Molekül, vorzugsweise ein Protein, das in der Lage ist, die nachzuweisende Substanz mit hoher Affinität zu binden. Dieses Protein wird im Folgenden als "Rezeptor" bezeichnet. Ein erfindungsgemäß hergestellter biochemischer Sensor enthält wenigstens einen Rezeptor sowie die Moleküle, die in der den Rezeptor/die Rezeptoren stabilisierenden Zelllysatfraktion enthalten sind. Der Fachmann weiß, dass ein biochemischer Sensor darüber hinaus weitere Moleküle enthalten kann.

Die Bindung des Analyten an den Rezeptor ermöglicht den qualitativen und bevorzugt auch den quantitativen Nachweis des Analyten in einer Probe. Unter qualitativem Nachweis wird die Feststellung der An- oder Abwesenheit des Analyten in der Probe verstanden. Der Fachmann weiß, dass der qualitative Nachweis abhängig vom verwendeten Sensor eine untere Nachweisgrenze hat, d.h. die Probe muss eine bestimmte Mindestkonzentration des Analyten aufweisen, damit dessen Anwesenheit festgestellt werden kann. Ein quantitativer Nachweis des Analyten liefert zusätzlich noch Informationen über die Menge oder Konzentration des Analyten in der Probe. Um die Wechselwirkung zwischen Rezeptor und Analyten messbar zu machen ist es bevorzugt, das verwendete Protein chemisch zu modifizieren. Besonders bevorzugt ist die Modifikation eines isolierten Proteins durch Markierung mit Fluoreszenzfarbstoffen.

Der Begriff "Probe" bezeichnet jede Flüssigkeit, in der der Analyt nachgewiesen werden soll. Bevorzugt sind Blut, Plasma, Serum, Tränenflüssigkeit, Gewebsflüssigkeit und Urin, besonders bevorzugt ist die Gewebsflüssigkeit unter der Bindehaut des Auges. Gleichfalls bevorzugt sind Gewebeproben. Die Probe liegt bevorzugt als aus dem Körper des Patienten isolierte Probe vor. Stärker bevorzugt liegt die Probe an ihrem natürlichen Ort vor. In diesem Fall wird der Sensor bevorzugt in den Körper des Patienten eingebracht und wird direkt an dem Ort eingesetzt, an dem sich die Probe befindet.

In einer bevorzugten Ausführungsform der Erfindung enthält der biochemische Sensor Concanavalin A als Rezeptor. Solche biochemischen Rezeptoren können zur Bestimmung der Glucose-Konzentration in Proben eingesetzt werden, da Concanavalin A Glucose bindet. In diesem Fall enthält der biochemische Sensor bevorzugt Hydrogele zusätzlich zum Rezeptor und der den Rezeptor stabilisierenden Zelllysatfraktion. Bevorzugt sind Hydrogele aus synthetischen Molekülen, Biomolekülen oder modifizierten Biomolekülen. Bevorzugte synthetische Moleküle sind Polyacrylate, Polyacrylamide und Polyvinylalkohole. Bevorzugte Biomoleküle sind Gelatine, Carageenan, Agarose, Amylose, Amylopectin, Alginate, Gellan, Cellulose und Cellulose-Derivate. Besonders bevorzugt sind Polyvinylalkohole und Alginat. Wenn der biochemische Sensor besagte Hydrogele enthält, liegen Rezeptor und Analyt in heterogener Phase vor.

In einer besonders bevorzugten Ausfiihrungsform enthält der biochemische Sensor Hydrogelpartikel, in denen die das isolierte Protein stabilisierende Zelllysatfraktion, Dextran und Concanavalin A enthalten sind.

Weiterhin betrifft die vorliegende Erfindung die Verwendung einer erfindungsgemäßen ein isoliertes Protein stabilisierenden Zelllysatfraktion oder einer Präcanavalin enthaltenden Zubereitung zur Herstellung eines biochemischen Sensors.

Schließlich betrifft die vorliegende Erfindung einen biochemischen Sensor umfassend eine erfindungsgemäße ein isoliertes Protein stabilisierende Zelllysatfraktion oder eine Präcanavalin enthaltende Zubereitung.

Die folgenden Ausführungsbeispiele dienen nur dazu, die Erfindung zu illustrieren. Sie sollen den Gegenstand der Patentansprüche in keiner Weise beschränken.

### Beispiele:

### Beispiel 1: Gewinnung der nichtbindenden Fraktion aus Schwertbohnen

300 g Schwertbohnenmehl (Canavalia ensiformis) werden in 100 mM Puffer suspendiert und zur Entfernung von Membranbestandteilen filtriert. Insgesamt werden 30 g Protein auf eine Superdex 200-Säule aufgetragen und die nicht an das Säulenmaterial bindende Proteinfraktion wird gesammelt. Insgesamt erhält man 3 Liter nichtbindende Fraktion (nbF). Die Lösung wird gegen Wasser dialysiert. Anschließend erfolgt eine Gefriertrocknung (Lyophilisation). Die lyophilisierte Substanz kann im Arbeitspuffer aufgenommen werden.

### Beispiel 2: Aufreinigung von Präcanavalin

### Extraktion einer Rohfraktion Präcanavalin aus der nichtbindenden Fraktion

8,0 g dialysiertes und gefriergetrocknetes nbF wird mit Fällungspuffer (50 mM Na-Acetat, pH 4,4) versetzt, inkubiert und anschließend zentrifugiert. Das Sediment der Fällung wird mit Wasser gewaschen und wiederum zentrifugiert. Das Pellet wird in 800 ml Salz-Hydrogenphosphatlösung (1% (w/v) NaCl + 0,1% (w/v) K₂HPO₄, pH 7,0) gelöst. Der Überstand nach Zentrifugation wird gesammelt. Das Sediment wird in 500 ml 5%iger Kochsalzlösung gelöst und anschließend zentrifugiert. Die vereinigten Überstände der Salzextraktionen werden gegen Aqua bidest dialysiert. Das Dialysat wird zentrifugiert . Das Sediment wird verworfen und der Überstand mit 1N Essigsäure auf pH 5,1 zur Präzipitation eingestellt. Der Präzipitationsansatz wird über Nacht inkubiert und am nächsten Tag zentrifugiert. Das Sediment wird in 50 ml 0,01N NaOH aufgenommen und mit 6 ml 0,1N NaOH versetzt, so dass eine nahezu klare Lösung vorliegt. Der pH-Wert wird überprüft uns sollte nicht mehr als pH 8,0 betragen. Der Ansatz wird bei 4°C über Nacht inkubiert. Der Säure-Base Extraktionsschritt wird einmal wiederholt. Das zuletzt erhaltene basische Präcanavalinextrakt wird gegen 20 mM Tris-Puffer, 100 mM NaCl, pH 7,2 dialysiert.

### Reinigung von Präcanavalin

Die Reinigung des Präcanavalins erfolgt über DEAE-Anionentauscherchromatographie (DEAE Sepharose FF). Das Protein wird auf die mit Auftragspuffer (20 mM Tris-Puffer, 100 mM NaCl, pH 7,2) äquilibierte Säule aufgetragen, mit Puffer nachgewaschen bis die Basis-Absorptionslinie bei 280 nm stabil ist und anschließend eluiert (Elutionspuffer: 20 mM Tris-Puffer, 1 M NaCl, pH 7,2). Die Elution erfolgt mit einem Salzgradienten von 0-100%. Nach der Analytik der Elutionsfraktionen mittels SDS-PAGE erfolgt die Vereinigung der reinsten Fraktionen. Das gereinigte Präcanavalin wird gegen Aqua bidest über Nacht dialysiert und dann gefriergetrocknet.

### Beispiel 3: Herstellung von Hydrogelpartikeln mit nichtbindender Fraktion (oder Präcanavalin)

2 g Alginsäure-Natriumsalz und 8 g nichtbindende Fraktion (oder Präcanavalin) werden in einem 250 ml Erlenmeyerkolben in 200 g Wasser unter Rühren gelöst. In einem 5L Becher werden 66,2 g CaCl2 x 2H2O in 4931,3 g Wasser gelöst.

Die Alginat/Protein-Lösung wird über eine Pumpe in eine Zwei-Stoffdüse gefördert. Gleichzeitig wird am zweiten Eingang der Düse Druckluft angelegt, sodass die Alginat/Protein-Lösung in feine Tröpfchen zerstäubt wird. Die Tröpfchen werden durch die Luftströmung in ein Ultraschallbad mit der Calciumchlorid Lösung getragen, wo sie gelieren und zu Boden sinken. Die gelierten Kugeln werden anschließend gesammelt und ggf. autoklaviert.

### Beispiel 4: In vitro Stabilitätstest von Glucosesensoren

Glucosesensoren werden in jeweils 1 ml physiologischem Puffer bei 37°C gelagert. Nach entsprechender Lagerdauer wird der Glucosesensor entnommen und das Fluoreszenzspektrum bei verschiedenen Glucosekonzentrationen bestimmt. Die Veränderung der Fluoreszenzintensitäten mit steigendem Glucosegehalt dient als Maß für die Güte des Glucosesensors. Sensoren verschiedener Lagerdauern werden mit ungelagerten Sensoren verglichen und die absolute und prozentuale Abnahme der Reaktion auf Glucose über die Lagerdauer bestimmt.

Tabelle 1 zeigt vergleichend die Sensorantwort von mit nichtbindender Fraktion und mit Präcanavalin stabilisierten Sensoren im physiologischen Glucosebereich von 50 bis 250 mg/dL, ausgedrückt als prozentuale Signaländerung pro mg/dL Erhöhung der Glucosekonzentration. Nichtbindende Fraktion und Präcanavalin liefern identische Ergebnisse.

**Tabelle 1: Lagerfähigkeit stabilisierter Glucosesensoren**

| **Stabilisator** | **Sensorresponse im physiologischen Glucosebereich zwischen 50 und 250 mg/dL Glucose nach verschiedenen Lagerdauern [ Signaländerung / mg/dL Glucose]** | | |
|---|---|---|---|
| | 14 Tage | 60 Tage | 120 Tage |
| nichtbindende Fraktion | 0,46 | 0,29 | 0,22 |
| Präcanavalin | 0,44 | 0,29 | 0,20 |

### Beispiel 5: Ergebnis des Stabilitätstests mit verschiedenen möglichen Stabilisatoren relativ zu nicht stabilisiertem Sensor

Tabelle 2 zeigt Werte nach 120 Tagen Lagerung unter physiologischen Bedingungen. Während bekannte Stabilisatoren wie BSA, HSA das Sensorsystem nicht stabilisieren, wird durch nichtbindende Fraktion aus Bohnenmehlextrakt (auch autoklaviert) eine deutliche Stabilisierung erzielt.

**Tabelle 2: Vergleich verschiedener Stabilisatoren**

| **Stabilisator** | **Verbesserung Sensorresponse nach 120 Tagen Lagerung im Vergleich zu nicht stabilisiertem System** |
|---|---|
| HSA | 2 % |
| BSA | -3 % |
| nichtbindende Fraktion | 41 % |
| autoklavierte nichtbindende Fraktion | 27 % |

## Patentansprüche

1. Verfahren zur Bereitstellung einer ein isoliertes Protein stabilisierenden Zelllysatfraktion umfassend die Schritte
a) Gewinnung des Zelllysats aus den Zellen; und
b) Abtrennung des zu isolierenden Proteins aus dem Zelllysat.

2. Verfahren gemäß Anspruch 1, wobei das isolierte Protein und die das isolierte Protein stabilisierende Zelllysatfraktion aus *Canavalia ensiformis* gewonnen werden.

3. Verfahren gemäß Anspruch 2, wobei die das isolierte Protein stabilisierende Zelllysatfraktion Präcanavalin enthält.

4. Verfahren nach einem der Ansprüche 1 bis 3, wobei die das isolierte Protein stabilisierende Zelllysatfraktion nach der Abtrennung des isolierten Proteins weiter aufgearbeitet wird.

5. Verwendung einer nach einem der Ansprüche 1 bis 4 gewonnen Zelllysatfraktion zur Stabilisierung eines isolierten Proteins.

6. Verwendung nach Anspruch 5, wobei das Protein ein Rezeptor ist.

7. Verwendung nach Anspruch 6, wobei der Rezeptor Concanavalin A ist.

8. Verwendung nach einem der Ansprüche 5 bis 7, wobei das isolierte Protein Bestandteil eines biochemischen Sensors ist.

9. Biochemischer Sensor umfassend eine nach einem der Ansprüche 1 bis 4 hergestellte ein isoliertes Protein stabilisierende Zelllysatfraktion.

10. Biochemischer Sensor nach Anspruch 9, wobei der Rezeptor Concanavalin A ist.

11. Biochemischer Sensor nach Anspruch 9 oder 10, wobei die das isolierte Protein stabilisierende Zelllysatfraktion, Dextran und Concanavalin A in Hydrogelpartikeln enthalten sind.


SEQUENCE LISTING

<110> EyeSense AG

<120> Stabilisierung von Biosensoren für in vivo Anwendungen

<130> ES66613EP

<160> 3

<170> PatentIn version 3.5

<210> 1
<211> 1403
<212> DNA
<213> Canavalia ensiformis

<400> 1
attttgtatt tagtaaacca atatggcttt ttctgcgcga tttccactat ggttattgct 60

gggagttgtt ttgcttgctt cagtttctgc gtcgtttgcg cactcgggac acagtggagg 120

agaagcagag gacgagagtg aagagtcacg ggcacaaaat aacccgtatc tctttaggtc 180

caacaagttc ctcactctct tcaagaacca acacggttct cttcgtctcc tccaaaggtt 240

caacgaagac accgagaaac tggagaatct tcgagactac cgagttcttg aatattgctc 300

caaaccaaac accctccttc tccctcacca ctccgattct gatcttctcg tccttgtcct 360

cgagggacaa gccatacttg ttttggtgaa ccctgacggc agagacactt acaaacttga 420

ccaaggcgat gctatcaaaa tccaagcagg gacccctttc tatctcatta acccagacaa 480

caaccagaac ctcagaatat taaagttcgc cataaccttc aggagaccgg gcacagtcga 540

ggatttcttc ctatctagca ctaaaagact gccatcctac ctgagtgcgt tcagcaagaa 600

ttttctagag gcctcctacg attccccata tgacgagata gagcagactc tgttgcaaga 660

agaacaagag ggagtgatag tcaaaatgcc aaaggatcag atccaggaaa taagcaaaca 720

tgcccaatct agctccagaa aaacactttc ttcccaagat aaaccattta acttgagaag 780

ccgagacccc atctattcca acaactatgg caagttatat gagatcactc cagagaaaaa 840

ctcacagcta cgggacttgg atatcctcct caattgttta caaatgaatg agggagctct 900

tttgtgccca cactacaatt caagggccac agtcatactg gtggctaatg aaggaagagc 960

agaggttgag ttggtgggtc tagaacagca acaacagcaa ggattagaaa gtatgcaact 1020

gcggaggtac gctgccacgt tatctgaagg cgatataatc gtaattccct cgtcttttcc 1080

ggttgccctc aaagctgctt cagatctaaa tatggttggg attggtgtca atgctgaaaa 1140

taacgagagg aacttccttg caggtcacaa agagaacgtg ataaggcaga tacctaggca 1200

agtgagtgat cttacattcc ctggatctgg tgaagaggtt gaggagttat tagagaatca 1260

aaaggaatcc tactttgtgg atggtcagcc taggcatatt gacgctggtg gaaaggctag 1320

aagggcccat ctgcctaatc ttttccgcac tttttactga ataaactatc taagttacta 1380

aataaaatgc tgtaaaagca aag 1403

<210> 2
<211> 445

<212>  PRT
<213>  Canavalia ensiformis

<400>  2

Met Ala Phe Ser Ala Arg Phe Pro Leu Trp Leu Leu Leu Gly Val Val
1               5                   10                  15

Leu Leu Ala Ser Val Ser Ala Ser Phe Ala His Ser Gly His Ser Gly
            20                  25                  30

Gly Glu Ala Glu Asp Glu Ser Glu Glu Ser Arg Ala Gln Asn Asn Pro
        35                  40                  45

Tyr Leu Phe Arg Ser Asn Lys Phe Leu Thr Leu Phe Lys Asn Gln His
    50                  55                  60

Gly Ser Leu Arg Leu Leu Gln Arg Phe Asn Glu Asp Thr Glu Lys Leu
65                  70                  75                  80

Glu Asn Leu Arg Asp Tyr Arg Val Leu Glu Tyr Cys Ser Lys Pro Asn
                85                  90                  95

Thr Leu Leu Leu Pro His His Ser Asp Ser Asp Leu Leu Val Leu Val
            100                 105                 110

Leu Glu Gly Gln Ala Ile Leu Val Leu Val Asn Pro Asp Gly Arg Asp
        115                 120                 125

Thr Tyr Lys Leu Asp Gln Gly Asp Ala Ile Lys Ile Gln Ala Gly Thr
    130                 135                 140

Pro Phe Tyr Leu Ile Asn Pro Asp Asn Asn Gln Asn Leu Arg Ile Leu
145                 150                 155                 160

Lys Phe Ala Ile Thr Phe Arg Arg Pro Gly Thr Val Glu Asp Phe Phe
                165                 170                 175

Leu Ser Ser Thr Lys Arg Leu Pro Ser Tyr Leu Ser Ala Phe Ser Lys
            180                 185                 190

Asn Phe Leu Glu Ala Ser Tyr Asp Ser Pro Tyr Asp Glu Ile Glu Gln
        195                 200                 205

Thr Leu Leu Gln Glu Glu Gln Glu Gly Val Ile Val Lys Met Pro Lys
    210                 215                 220

Asp Gln Ile Gln Glu Ile Ser Lys His Ala Gln Ser Ser Ser Arg Lys
225                 230                 235                 240

Thr Leu Ser Ser Gln Asp Lys Pro Phe Asn Leu Arg Ser Arg Asp Pro
                245                 250                 255

Ile Tyr Ser Asn Asn Tyr Gly Lys Leu Tyr Glu Ile Thr Pro Glu Lys
            260                 265                 270

Asn Ser Gln Leu Arg Asp Leu Asp Ile Leu Leu Asn Cys Leu Gln Met
        275                 280                 285

Asn Glu Gly Ala Leu Phe Val Pro His Tyr Asn Ser Arg Ala Thr Val
    290                 295                 300

Ile Leu Val Ala Asn Glu Gly Arg Ala Glu Val Glu Leu Val Gly Leu
305                 310                 315                 320

Glu Gln Gln Gln Gln Gln Gly Leu Glu Ser Met Gln Leu Arg Arg Tyr
                325                 330                 335

Ala Ala Thr Leu Ser Glu Gly Asp Ile Ile Val Ile Pro Ser Ser Phe
            340                 345                 350

Pro Val Ala Leu Lys Ala Ala Ser Asp Leu Asn Met Val Gly Ile Gly
        355                 360                 365

Val Asn Ala Glu Asn Asn Glu Arg Asn Phe Leu Ala Gly His Lys Glu
    370                 375                 380

Asn Val Ile Arg Gln Ile Pro Arg Gln Val Ser Asp Leu Thr Phe Pro
385                 390                 395                 400

Gly Ser Gly Glu Glu Val Glu Glu Leu Leu Glu Asn Gln Lys Glu Ser
                405                 410                 415

Tyr Phe Val Asp Gly Gln Pro Arg His Ile Asp Ala Gly Gly Lys Ala
            420                 425                 430

Arg Arg Ala His Leu Pro Asn Leu Phe Arg Thr Phe Tyr
        435                 440                 445


<210>  3
<211>  290
<212>  PRT
<213>  Canavalia ensiformis

<400>  3

Met Ala Ile Ser Lys Lys Ser Ser Leu Phe Leu Pro Ile Phe Thr Phe
1               5                   10                  15

Ile Thr Met Phe Leu Met Val Val Asn Lys Val Ser Ser Ser Thr His
            20                  25                  30

Glu Thr Asn Ala Leu His Phe Met Phe Asn Gln Phe Ser Lys Asp Gln
        35                  40                  45

Lys Asp Leu Ile Leu Gln Gly Asp Ala Thr Thr Gly Thr Asp Gly Asn
    50                  55                  60

Leu Glu Leu Thr Arg Val Ser Ser Asn Gly Ser Pro Gln Gly Ser Ser
65                  70                  75                  80

Val Gly Arg Ala Leu Phe Tyr Ala Pro Val His Ile Trp Glu Ser Ser
                85                  90                  95

Ala Val Val Ala Ser Phe Glu Ala Thr Phe Thr Phe Leu Ile Lys Ser
            100                 105                 110

Pro Asp Ser His Pro Ala Asp Gly Ile Ala Phe Phe Ile Ser Asn Ile
        115                 120                 125

Asp Ser Ser Ile Pro Ser Gly Ser Thr Gly Arg Leu Leu Gly Leu Phe
    130                 135                 140

Pro Asp Ala Asn Val Ile Arg Asn Ser Thr Thr Ile Asp Phe Asn Ala
145                 150                 155                 160

Ala Tyr Asn Ala Asp Thr Ile Val Ala Val Glu Leu Asp Thr Tyr Pro
                165                 170                 175

Asn Thr Asp Ile Gly Asp Pro Ser Tyr Pro His Ile Gly Ile Asp Ile
            180                 185                 190

Lys Ser Val Arg Ser Lys Lys Thr Ala Lys Trp Asn Met Gln Asn Gly
        195                 200                 205

Lys Val Gly Thr Ala His Ile Ile Tyr Asn Ser Val Asp Lys Arg Leu
    210                 215                 220

Ser Ala Val Val Ser Tyr Pro Asn Ala Asp Ser Ala Thr Val Ser Tyr
225                 230                 235                 240

Asp Val Asp Leu Asp Asn Val Leu Pro Glu Trp Val Arg Val Gly Leu
                245                 250                 255

Ser Ala Ser Thr Gly Leu Tyr Lys Glu Thr Asn Thr Ile Leu Ser Trp
            260                 265                 270

Ser Phe Thr Ser Lys Leu Lys Ser Asn Glu Ile Pro Asp Ile Ala Thr
        275                 280                 285

Val Val
    290